# EUROPEAN PATENT SPECIFICATION

(11) **EP 0 616 536 B1**
(45) Date of publication and mention of the grant of the patent: **17.03.1999**
(21) Application number: 93900930.4
(22) Date of filing: 04.12.1992
(51) Int. Cl.: A61K 38/00, A61K 38/28

(54) **Use of decorin or biglycan for the manufacture of a medicament in the treatment of diabetes-associated pathology**
Verwendung von Decorin oder Biglycan zur Herstellung eines Medikaments für die Behandlung von Diabetes-bedingter Zustände
Utilisation de décorine ou de biglycane pour la fabrication d'un médicament destiné au traitement de la pathologie associée au diabète

(30) Priority: 04.12.1991 US 803285
(43) Date of publication of application: 28.09.1994
(73) Proprietor: LA JOLLA CANCER RESEARCH FOUNDATION, La Jolla, CA 92037 (US)
(72) Inventor: RUOSLAHTI, Erkki, I., Rancho Santa Fe, CA 92067 (US); BORDER, Wayne, A., Salt Lake City, UT 84124 (US)
(74) Representative: UEXKÜLL & STOLBERG
(86) International application number: US9210550
(87) International publication number: WO9310808

(56) References cited:
- WO-A-91/04748
- WO-A-91/10727
- Dialog Information Services, File 154, Medline, Dialog accession no. 07764951, Medline accession no.91283951, Border WA et al: "Transforming growth factor-beta 1 induces extracellular matrix formation in glomerulonephritis", Cell Differ Dev Dec 2 1990,32 (3) p 425-31
- NATURE, Volume 346, July 1990, Wayne A. Border et al, "Suppression of experimental glomerulonephritis by antiserum against transforming growth factor beta1"
- Dialog Information Services, File 154, Medline, Dialog accession no. 06912696, Medline accession no.89214696, Connor TB Jr et al: "Correlation of fibro-sis and transforming growth factor-beta type 2 le- vels in the eye", J Clin Invest May 1989, 83 (5) p 1661-6

## Description

### BACKGROUND OF INVENTION

Various pathologies are characterized by a deleterious accumulation of extracellular matrix materials. For example, in progressive glomerular disease, extracellular matrix accumulates in the mesangium or along the glomerular basement membrane, eventually causing end-stage disease and uremia. Similarly, adult or acute respiratory distress syndrome (ARDS) involves the accumulation of matrix materials in the lung, while cirrhosis of the liver is characterized by deleterious matrix accumulation evidenced by scarring in the liver.

Diabetes is now the most common cause of progressive kidney failure and also leads to conditions characterized by the deleterious accumulation of extracellular matrix components. The introduction of insulin revolutionized the treatment of diabetes and dramatically lengthened patient survival. However, insulin has not been effective in preventing such serious complications as diabetic nephropathy. Although strict control of blood glucose, treatment of hypertension, and restriction of dietary protein may slow the rate at which kidney function is lost, there is a steady progression to end-stage disease.

The central pathological feature of diabetic nephropathy is accumulation of extracellular matrix within the glomeruli. The factors in the diabetic milieu responsible for extracellular matrix accumulation are poorly understood.

Extracellular matrix is a mixture of proteoglycans, glycoproteins and collagens assembled into a complex superstructure. Although a variety of immunologic, hemodynamic and toxic factors have been used experimentally to induce glomerular disease, none of these factors has been shown to directly influence synthesis or degradation of extracellular matrix components. Thus it seems likely that there is another intervening process between acute cell injury and buildup of glomerular extracellular matrix.

Thus, a need exists to determine the factors which regulate deleterious accumulation of matrix components in pathological states such as kidney disease. Further, there exists a need to control such factors so as to prevent, limit or treat pathogenic conditions which include inappropriate matrix accumulation. The present invention satisfies these needs and provides related advantages as well.

### SUMMARY OF THE INVENTION

The present invention relates to the use of decorin or biglycan for the preparation of a medicament for preventing or treating a pathology characterized by an accumulation of extracellular matrix in a tissue, wherein said pathology is diabetes-associated pathology. In a particular embodiment of the invention, the pathology is diabetic nephropathy.

The invention further relates to the use of insulin and decorin or biglycan for the preparation of a medicament for treating diabetic nephropathy. The invention is further directed to a composition comprising insulin and decorin or biglycan.

### BRIEF DESCRIPTION OF THE DRAWINGS

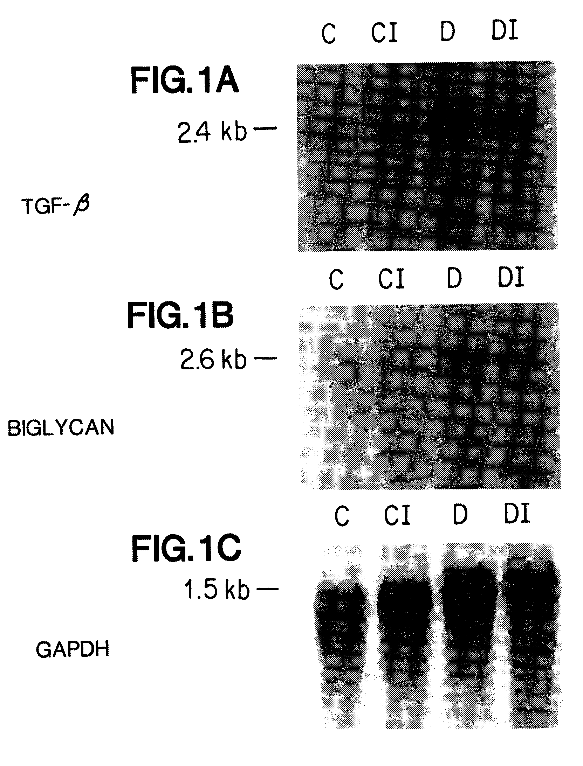

Figure 1 shows the results of northern blotting of mRNA, isolated from rat glomeruli, hybridized with a cDNA probe to (A) TGF-β1, (B) biglycan, and (C) glyceraldehyde-3-dehydrogenase (GAPDH), an enzyme constitutively expressed in the rat glomeruli. mRNA was prepared from glomeruli isolated from control rats (C), control rats treated with insulin (CI), diabetic rats (D) and diabetic rats treated with insulin (DI) 15 weeks after induction of diabetes. Molecular weight markers are shown to the left.

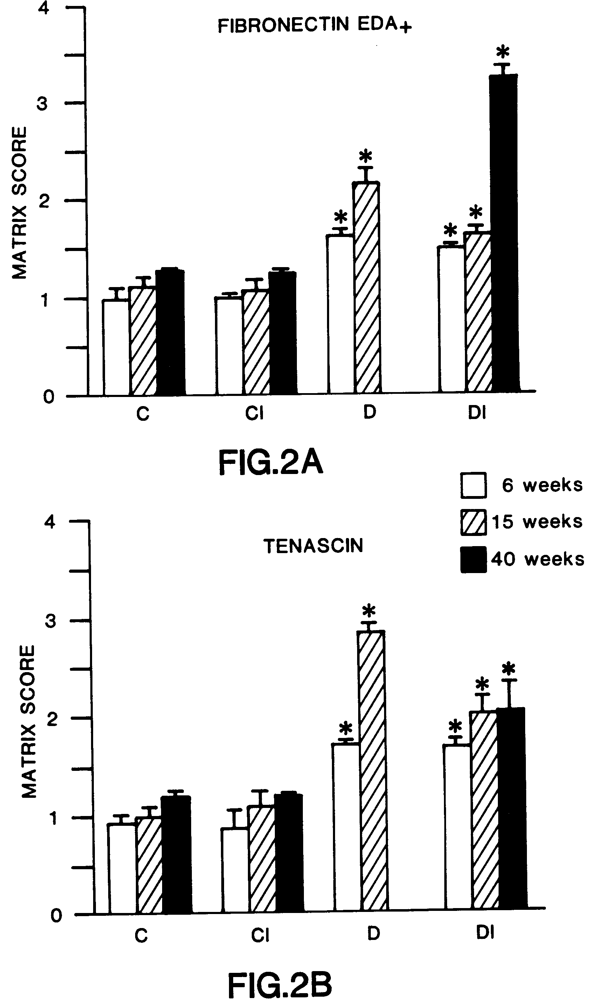

Figure 2 shows the quantitation of an alternately spliced form of fibronection, fibronection EDA+ (Figure 2A), and tenascin (Figure 2B), in glomeruli of control and diabetic rats. Asterisk denotes p<0.01, for diabetic rats (D) and diabetic rats treated with insulin (DI) compared to normal controls (C) at the same time point. Normal control rats treated with insulin (CI) are included for comparison. Diabetic animals not treated with insulin did not survive beyond 37 weeks and are thus absent from the data at 40 weeks. Values are mean ± s.d.

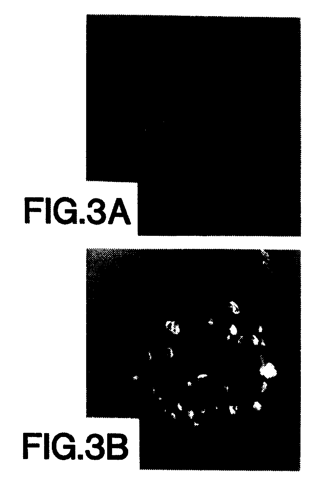

Figure 3 shows immunofluorescence micrographs of glomeruli stained with anti-TGF-β1 antibody. (A) Staining of a glomerulus of a normal control rat. (B) Staining of a glomerulus from a diabetic rat, not treated with insulin, 15 weeks after induction of diabetes shows a striking increase in the number of cells positive for TGF-β1. The positive cells were identified as glomerular mesangial and epithelial cells. In the diabetic glomeruli there were significantly more positive cells per glomerulus, *32 ± 1 versus 25 ± 1, (values are mean ± s.e., *p<0.01) compared to glomeruli from the control rats. The photographs were taken under identical conditions with equal exposure times of 60 seconds. Magnification x 500.

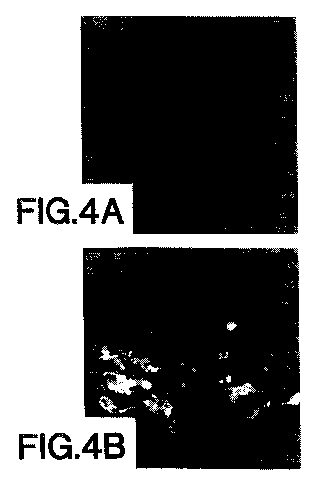

Figure 4 shows immunofluorescence micrographs of glomeruli stained with anti-fibronectin EDA+ antibody. (A) Glomerulus of a normal control rat, treated with insulin for 40 weeks shows faint staining. (B) Staining of a glomerulus from a rat with diabetes, treated with insulin for 40 weeks shows a marked increase in staining. The photographs were taken under identical conditions with equal exposure times of 60 seconds. Magnification x 500.

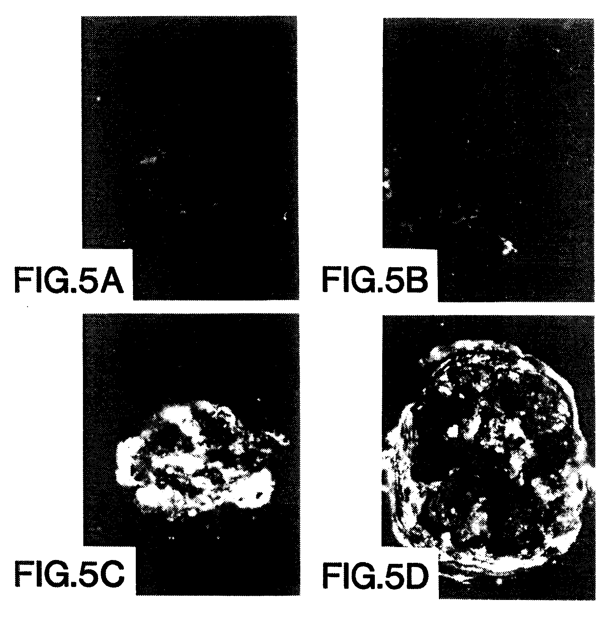

Figure 5 shows immunofluorescence of human glomeruli stained with anti-TGF-β1 antibody. (A) Faint staining of a glomerulus from a normal kidney and (B) from the kidney of a patient with minimal change disease. (C and D) Glomeruli from two patients with severe matrix accumulation characteristic of advanced diabetic glomerulosclerosis show bright staining of immunoreactive TGF-β1 protein. In another case of early diabetic nephropathy with less matrix accumulation, the staining pattern was intermediate between that seen in these cases of advanced human disease and that seen in the 15 week diabetic rats shown in Figure 3B, suggesting that the pattern of TGF-β1 staining follows a continuum with disease severity, initially showing distinct cellular staining and later increasing to such an extent that cellular staining is obscured. (D) This micrograph also shows staining in the epithelial (Bowman) capsule surrounding the glomerulus. The photographs were taken under identical conditions with equal exposure tees of 60 seconds. Magnification x 500.

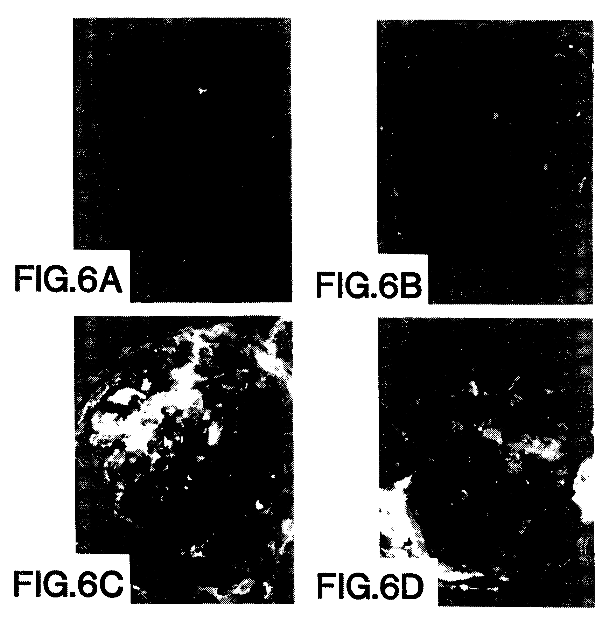

Figure 6 shows immunofluorescence of human glomeruli stained with anti-fibronectin EDA+ antibody. (A) Glomerulus from a normal kidney and (B) from a patient with minimal change disease are barely stained. (C and D) In contrast two patients with diabetic glomerulosclerosis show strong staining within the abnormal glomeruli and the surrounding (Bowman) capsules. The photographs were taken under identical conditions with equal exposure times of 60 seconds.

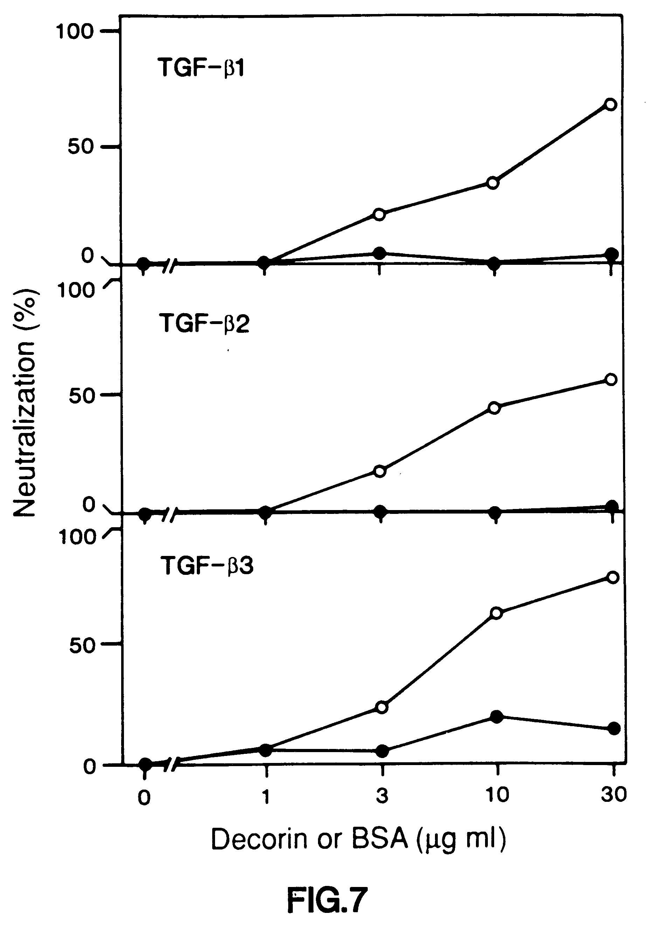

Figure 7 shows neutralization of the activity of TGF-β1, 2 and 3 by recombinant human decorin. A mink lung cell assay that measures growth inhibition was used to assay the ability of recombinant decorin to neutralize the activity of the TGF-β isoforms. [³H]Thymidine incorporation was determined in the presence of TGF-β and increasing concentrations of decorin (o) or bovine serum albumin (BSA) (●) as described by Yamaguchi et al., Nature 346:281 (1990), incorporated herein by reference. The data represent percent neutralization of TGF-β1-induced growth inhibition. [³H]Thymidine incorporation in the presence of neither TGF-β nor decorin is defined as 100% (125,000 cpm for TGF-β1 and TGF-β3; 107,000 cpm for TGF-β2). Incorporation in the presence of TGF-β but not decorin is defined as 0% (57,000 cpm for TGF-β1; 78,000 cpm for TGF-β2; 59,000 cpm for TGF-β3). Each point represents the mean of results from duplicate samples.

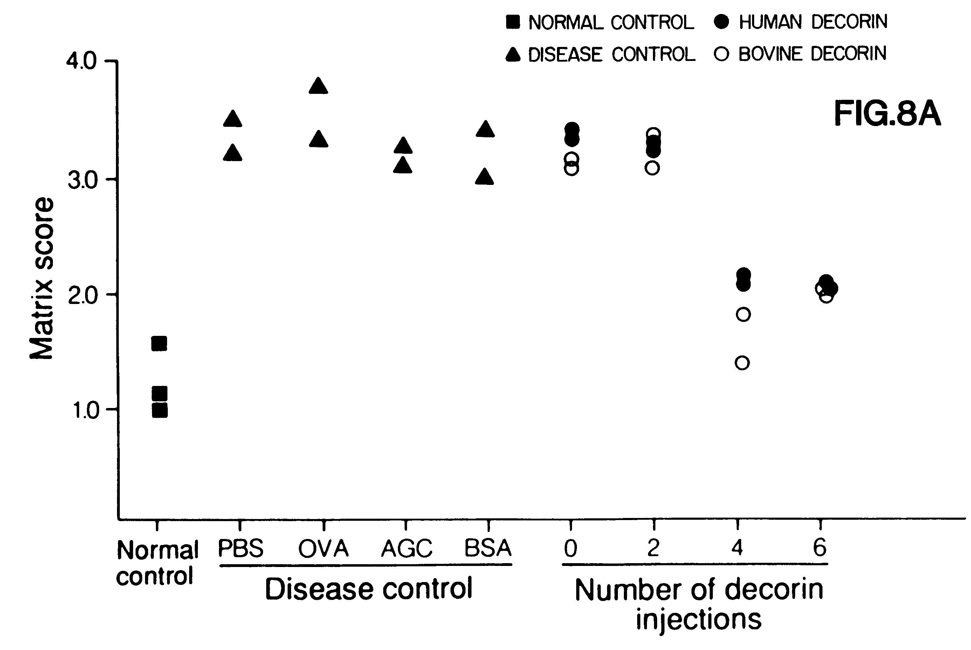

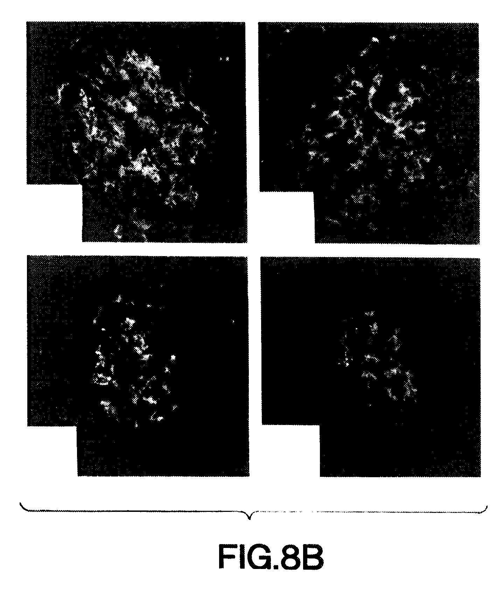

Figure 8 shows deposition of fibronectin in glomeruli of decorin-treated glomerulonephritic rats. (A) Quantitation of fibronectin staining in glomeruli from normal controls, control glomerulonephritic rats that received six injections of phosphate buffered saline (PBS), ovalbumin (OVA), aggrecan (AGC) or bovine serum albumin (BSA) or various amounts of decorin. Rats treated with four or six injections of decorin had significantly less fibronectin deposited in glomeruli than did the disease controls or rats that received no or two injections of decorin (p<0.01). (B) Immunofluorescence micrographs of glomeruli from glomerulonephritic rats stained with anti-fibronectin antibody. The rats were treated with no injections of decorin (ND) (i.e. PBS alone) or injections of decorin for two days (2D), four days (4D) or six days (6D). The maximum standard error of the mean for glomerular scores in any animal was 0.16 indicating low interglomerular variability. Matrix scores of the illustrated glomeruli are: ND, 3.5; 2D, 3.5; 4D, 2.0; 5D, 2.0. The photographs were taken under identical conditions with exposure times of 40 seconds. Magnification x 500.

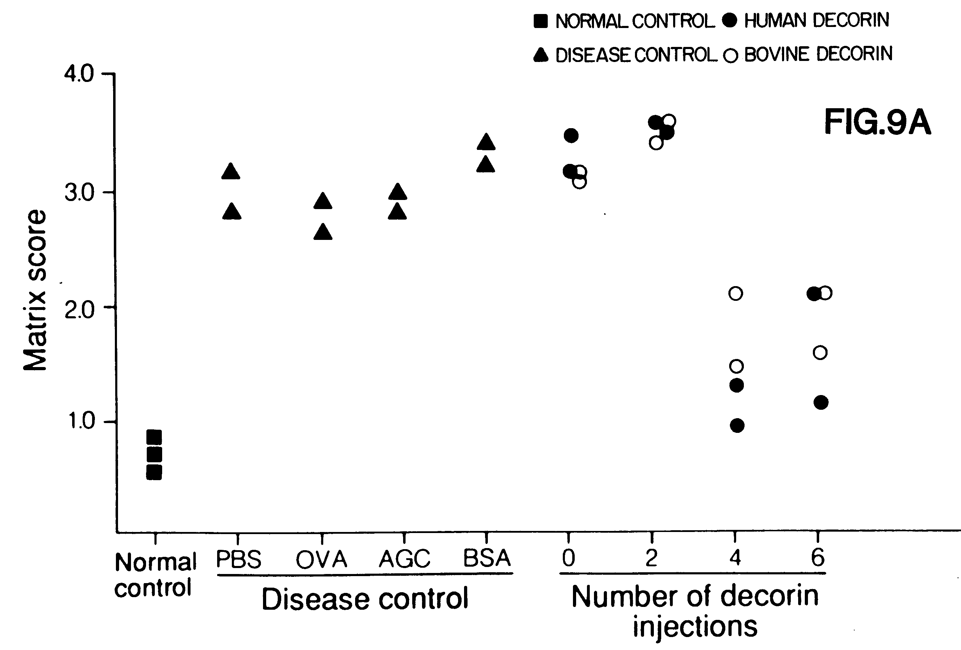

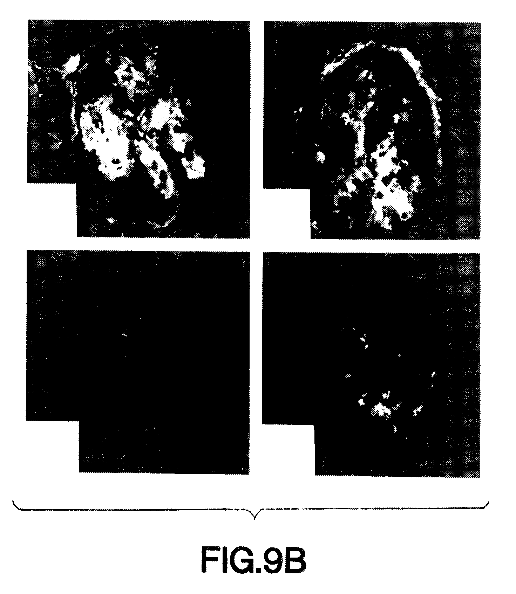

Figure 9 shows deposition of EDA+ fibronectin and tenascin in glomeruli of decorin-treated glomerulonephritic rats. (A) Quantitation of EDA+ fibronectin staining in glomeruli. Symbols are as described in Figure 8A. Rats that received four or six injections of decorin were significantly protected from the deposition of EDA+ fibronectin compared to all other glomerulonephritic groups (p<0.01). (B) Immunofluorescence micrographs of glomeruli from glomerulonephritic rats stained with anti-EDA fibronectin antibody. Symbols are as described in Figure 8B. The maximum standard error of the mean for glomerular scores in any animal was 0.11 indicating low interglomerular variability. Matrix scores of the illustrated glomeruli are: ND, 4.0; 2D, 3.5; 4D, 1.0; 6D. 1.5. The photographs were taken under identical conditions with exposure times of 60 seconds. Magnification x 500. Immunofluorescence micrographs of glomeruli from glomerulonephritic rats stained with anti-tenascin antibody also were obtained. Rats were treated with four or six injections of decorin and had less tenascin in glomeruli than did rats treated with no or two injections of decorin. Both EDA+ fibronectin and tenascin were greatly increased in nephritic glomeruli in rats treated with no or two injections of decorin.

### DETAILED DESCRIPTION OF THE INVENTION

The invention provides a method to inhibit the accumulation of extracellular matrix components in a tissue by contacting the tissue with an agent that suppresses the activity of transforming growth factor β (TGF-β). The invention also provides a method for treating pathologies characterized by an accumulation of extracellular matrix in a tissue by contacting the tissue with an effective amount of agent that suppresses the extracellular matrix producing activity of TGF-β. According to the present invention, the pathology is diabetes-associated pathology, in particular diabetic nephropathy, and the agent is decorin or biglycan.

The agent can be decorin, an analog thereof, or its functional equivalent. As used herein, "decorin" refers to a proteoglycan having substantially the structural characteristics attributed to it in Krusius and Ruoslahti, PNAS (USA) 83:7638 (1986). Human fibroblast decorin has substantially the amino acid sequence presented in Krusius and Ruoslahti, supra. "Decorin" refers both to the native composition and to modifications thereof which substantially retain the functional characteristics, such as decorin fragments. Decorin core protein refers to decorin that no longer is substantially substituted with glycosaminoglycan and is included in the definition of decorin. Decorin can be rendered glycosaminoglycan-free by enzymatic treatment, mutation or other means, such as by producing recombinant decorin in cells incapable of attaching glycosaminoglycan chains to a core protein.

Functional equivalents of decorin include modifications of decorin that retain its functional characteristics and molecules that are homologous to decorin, such as biglycan and fibromodulin, for example, that have the similar functional activity of decorin. Modifications can include, for example, the addition of one or more side chains that do not interfere with the functional activity of the decorin core protein.

This invention, in addition to the above listed agents, is intended to encompass the use of homologs and analogs of such agents. In this context, homologs are molecules having substantial structural similarities to the above-described agents and analogs are molecules having substantial biological similarities regardless of structural similarities.

One of skill in the art can readily determine whether a functional equivalent, homolog or analog functions for the purpose of this invention by substituting it into the examples provided herein.

Contacting can be effected in vitro or in vivo. When the contacting is in vitro, the cells can be incubated with an effective amount of the agent. When the contacting is in vivo, the agent can be administered alone or with a carrier. Methods of administering are well known to those of skill in the art and include, but not limited to intravenously or intramuscularly. The agent can be administered continuously or intermittently.

An effective amount will vary depending on the pathology and the individual being treated. The methods of this invention are useful for treating or protecting mammals, most preferably humans.

However, these pathologies are merely representative and a person skilled in the art would readily recognize the method to be useful in any diabetes-associated pathology characterized by an accumulation of extracellular matrix.

Prior to the present invention, it was unknown that diabetes-associated pathology is caused by TGF-β induced accumulation of extracellular matrix in the tissue.

WO91/04748 discloses treatment of pathologies characterized by an accumulation of extracellular matrix in a tissue. The pathologies, however, can be selected from the group consisting of glomerulonephritis, adult respiratory distress syndrom, cirrhosis of the liver, fibrotic cancer, fibrosis of the lungs, artheroslerosis, post-myocardial infarction, cardiac fibrosis, post-angioplasty restenosis, renal interstitial fibrosis and scarring.

Border et al., Nature 346 (1990), 371-374, disclose suppression of experimental glomerulonephritis by antiserum against TGF-β1.

However, it was neither disclosed in nor obvious from the art that diabetes-associated pathology could be treated by contacting the tissue with a medicament containing decorin or biglycan or with a composition comprising insulin and decorin or biglycan.

A variety of growth factors have been suggested to play a role in extracellular matrix production. However, their influence on the pathological accumulation of matrix components has been unclear. The present invention is predicated on the discovery that tissues prone to pathological accumulation of matrix synthesize particular proteoglycans. Agents that inhibit TGF-β activity, such as antibodies reactive with TGF-β, block the stimulatory effect of TFG-β on proteoglycan production. In this respect, TGF-β is unique among growth factors tested, and thus manipulating this specific effect of TGF-β has utility in controlling or treating the inappropriate and undesirable accumulation of matrix components in various pathologies.

TGF-β is a multifunctional cytokine that plays an important role in regulation repair and regeneration following tissue injury. Three isoforms of TGF-β, TGF-β1, 2, and 3, are expressed in mammals and to date show similar properties in vitro. Platelets contain high concentrations of TGF-β, and upon degranulation at a site of injury, release TGF-β into the surrounding tissue. TGF-β then initiates a sequence of events that promotes healing including (1) chemoattraction of monocytes, neutrophils, and fibroblasts, (2) autoinduction of TGF-β production and stimulation of monocytes to secrete interleukin-1 (IL-1), tumor necrosis factor and other cytokines, (3) induction of angiogenesis and cell proliferation, (4) control of inflammation and cell toxicity by acting as a potent immunosuppressant and inhibitor of peroxide release, and (5) increased deposition of extracellular matrix.

The effect of TGF-β on extracellular matrix is a key feature of its functional activities. TGF-β stimulates the synthesis of individual matrix components such as fibronectin, collagens and proteoglycans and simultaneously blocks matrix degradation by decreasing the synthesis of proteases and increasing the levels of protease inhibitors as described in Edwards et al., EMBO 6:1899 (1987) and Laiho et al., J. Biol. Chem. 262:17467 (1987). TGF-β also increases the expression of integrins and changes their relative proportions on the surface of cells in a manner that could facilitate adhesion to matrix as reported in Ignotz & Massague, Cell 51:189 (1987).

The mature form of TGF-β is comprised of two identical chains, each of 112 amino acids. TGF-β is responsible for the increased synthesis of extracellular matrix observed in various pathologies. The amino acid sequence of TGF-β is as follows:
Ala Leu Asp Thr Asn Tyr Cys Phe Ser Ser Thr Glu Lys Asn Cys Cys Val Arg Gln Leu Tyr Ile Asp Phe Arg Lys Asp Leu Gly Trp Lys Trp Ile His Glu Pro Lys Gly Tyr His Ala Asn Phe Cys Leu Gly Pro Cys Pro Tyr Ile Trp Ser Leu Asp Thr Gln Tyr Ser Lys Val Leu Ala Leu Tyr Asn Gln His Asn Pro Gly Ala Ser Ala Ala Pro Cys Cys Val Pro Gln Ala Leu Glu Pro Leu Pro Ile Val Tyr Tyr Val Gly Arg Lys Pro Lys Val Glu Gln Leu Ser Asn Met Ile Val Arg Ser Cys Lys Cys Ser.

Specific kidney pathologies are characterized by an increased accumulation of extracellular matrix components. For example, mesangial cells are one of the cell types that make up kidney glomerulus. In the normal glomerulus, the mesangial cells are surrounded by extracellular matrix. An increase in the quantity of mesangial matrix, with or without mesangial hypercellularity, is the earliest histologic finding in many forms of glomerulonephritis and in diabetic nephropathy. Cultured mesangial cells are known to secrete several matrix components including proteoglycans, fibronectin, laminin, entactin, thrombospondin and collagen types I, III, IV and V. However, the exact composition and supermolecular organization of the mesangial matrix, as well as the factors that control its synthesis assembly and degradation, have been unknown.

To study factors controlling the composition of the mesangial matrix, mesangial cells in culture were treated with IL-1, PDGF, tumor necrosis factor (TNF) and TGF-β. Analysis of the culture media indicated that TGF-β increased the amount of two components, identified as the proteoglycans biglycan and decorin. PDGF, IL-1, and TNF had no significant effect over the control.

Glomerulonephritis can be induced by specific immunological injury to the mesangial cell. Glomeruli isolated from animals with mesangial cell injury show increased biglycan and decorin production. Moreover, conditioned media from cultured nephritic glomeruli stimulate biglycan and decorin synthesis by normal mesangial cells.

An equivalent stimulatory effect can be produced by the addition of exogenous TGF-β. Moreover, agents that block the effect of TGF-β, such as an anti-TGF-β antiserum, block the stimulatory effect of exogenous TGF-β. Such agents, including monoclonal or polyclonal antibodies, PDGF, Arg-Gly-Asp-containing peptides, decorin or its functional equivalents can be used to specifically control or treat deleterious matrix proteoglycan synthesis. Thus, such agents can be used to prevent any condition associated with extracellular matrix accumulation, for example scarring, or to treat pathologies characterized by an accumulation of extracellular matrix in a tissue by contacting the tissue with an agent which suppresses TGF-β activity.

The effects of decorin on TGF-β were studied using the glomerulonephritis model. Because decorin appears to be a natural regulator of TGF-β, it should be active in vivo in this model and, if so, it would be an ideal molecule to employ therapeutically to antagonize the action of TGF-β. Intravenous administration of recombinant human decorin and decorin purified from bovine tissues was used to test for the ability of the proteogylcan to suppress matrix accumulation in glomerulonephritic rat kidneys. The amount of fibronectin present in glomeruli was used quantitatively to assess the ability of decorin to block matrix deposition in glomerulonephritis. Fibronectin is an abundant protein in the normal mesangial matrix of the rat and man and is greatly increased in humans with mesangial proliferative glomerulonephritis. Courtoy et al., J. Cell. Biol. 87:691 (1980), Courtoy et al., J. Histochem. Cytochem. 30:874 (1982), Oomura et al., Virchows Archiv. A Pathol. Anat. 415:151 (1989), Border, Kidney Int. 34:419 (1988). By using fibronectin staining as a marker of matrix it was found that decorin significantly prevented the acute buildup of matrix that occurs by day seven in the rat glomerulonephritis model. EDA+ fibronectin and tenascin provided more specific markers for TGF-β activity and matrix accumulation, because these proteins are almost undetectable in normal adult rat kidney and because they are strongly induced by TGF-β. Both markers were elevated in the nephritic glomeruli and both were suppressed by decorin injections. Finally, that the effect on matrix buildup was truly therapeutic was indicated by a parallel reduction in proteinuria, a widely used indicator of glomerular damage in humans, as described by Ginsberg et al., N. Engl. J. Med. 309:1543 (1983), incorporated herein by reference.

Acute mesangial proliferative glomerulonephritis has also been induced in animal models by injecting rats with anti-thymocyte serum as described in U.S. Application Serial No. 07/416,656, which is incorporated by reference in its entirety. It was found that injured glomeruli express more TGF-β mRNA, synthesize more TGF-β protein, and secrete significantly more fibronectin and proteoglycans than do normal glomeruli. Injection of an antiserum capable of neutralizing the activity of TGF-β into the nephritic rats suppressed the production of matrix components by the glomeruli and prevented the buildup of mesangial matrix.

Development of diabetic nephropathy (i.e., diabetic kidney disease) is one of the most frequent and serious complications of diabetes and eventually occurs in nearly half of patients despite treatment with insulin. The dominant histological feature of diabetic nephropathy is the expansion of extracellular matrix in the mesangial areas of the glomeruli with resulting capillary occlusion and sclerosis. Expansion of the mesangial matrix strongly correlates with the clinical onset of proteinuria, hypertension, and kidney failure.

The similarities of the matrix abnormalities of glomerulonephritis and diabetic nephropathy, where accumulation of extracellular matrix becomes pathological, suggested the potential involvement of TGF-β in diabetic kidney disease. It is shown by the present invention that glomeruli of rats made diabetic by the injection of streptozotocin contain a marked increase in the expression of TGF-β1 mRNA and TGF-β1 protein. The glomeruli also showed increased deposition of an alternatively spliced form of the extracellular matrix proteins, fibronectin and tenascin. Both of these components are known to be induced by TGF-β. These results indicate that TGF-β also contributes to the development of diabetic nephropathy.

In studies pertaining to the present invention, the kidneys of rats that were made diabetic by the administration of streptozotocin, a chemical that produces insulin deficiency, were examined. The streptozotocin model has been used extensively because the animals develop kidney disease that resembles human diabetic nephropathy as described in Velasquez et al., FASEB 4:2850 (1990) and Mauer et al., Diabetes 25:850 (1976), both of which are incorporated herein by reference. Rats were divided into groups that received no treatment or were treated with daily injections of insulin to reduce the blood glucose as described in Example II. Normal, age-matched, male rats served as controls and were also separated into groups that received no treatment or were treated with the same insulin regimen as the diabetic rats. To assess the expression of TGF-β in diabetes, the level of TGF-β1 mRNA and TGF-β1 protein were examined in glomeruli of the diabetic and control rats. The mRNA analysis showed elevated levels of TGF-β1 mRNA in glomeruli of diabetic rats compared to controls (Figure 1A). The levels of TGF-β1 mRNA increased with time after onset of diabetes and were higher in untreated diabetic rats compared to rats that received insulin as shown in Table 1.

**TABLE 1**

| Levels of TGF-β1 mRNA and Biglycan mRNA in Glomeruli | | | | |
|---|---|---|---|---|
| Time studied | Control (group 1) | Control+ Insulin (group 2) | Diabetes (group 3) | Diabetes+ Insulin (group 4) |
| | Ratios of TGF-β1 mRNA to GAPDH mRNA | | | |
| 1 w | 1.0 | 1.0 | 1.2 | 1.2 |
| 6 w | 1.0 | 1.0 | 2.1 | 1.6 |
| 15 w | 1.0 | 1.1 | 3.4 | 1.9 |

| | Ratios of biglycan mRNA to GAPDH mRNA | | | |
|---|---|---|---|---|
| 1 w | 1.0 | 1.0 | 1.1 | 1.2 |
| 6 w | 1.0 | 1.1 | 1.9 | 1.3 |
| 15 w | 1.0 | 1.0 | 2.3 | 1.6 |

Staining of glomeruli with an antibody that is thought to recognize newly synthesized TGF-β1 revealed significantly more positive cells in diabetic rats than in controls (see Figures 3A and 3B). The TGF-β1 positive cells did not stain with markers of monocyte/macrophages or lymphocytes but did show staining patterns characteristic of glomerular mesangial and/or epithelial cells.

To learn if TGF-β expressed in glomeruli of diabetic rats was active in stimulating extracellular matrix production, such glomeruli were stained with antibodies that detect an alternatively spliced form of fibronectin (fibronectin EDA+) and tenascin. These matrix components are induced by TGF-β as described in Border et al., Kidney Int. 37:689 (1990) and Nakamura et al., Kidney Int. 41:1213 (1992). The matrix components also appear at sites of tissue repair, Wang et al., J. Biol. Chem. 266:15598 (1991) and Pearson et al., EMBO J. 7:2977 (1988). Both fibronectin EDA+ and tenascin have also been found to be significantly increased in glomeruli of diabetic rats as early as six weeks after development of hyperglycemia, at a time when kidney histology is normal by light microscopy, and to continue to accumulate with time (Figures 1A and 2B).

Interstitial collagen type III is found in sclerotic glomeruli of humans with diabetic nephropathy but not in normal human or rat glomeruli as described in Nerlich & Schleicher, Am J. Pathol. 139:889 (1991). Type III collagen was found deposited in the glomeruli of the diabetic rats at six, fifteen and forty weeks. TGF-β, when added to cultures of normal rat mesangial cells or epithelial cells, is known to induce the production of decorin and biglycan, proteoglycans capable of binding TGF-β and neutralizing its activity. Biglycan mRNA levels were tested because rat biglycan mRNA, unlike rat decorin mRNA, cross-hybridizes with the corresponding human cDNA probes. Elevated levels of biglycan mRNA were detected in the glomeruli of diabetic rats as shown in Figure 1B and summarized in Table 1. These results further show that TGF-β is actively and specifically inducing matrix component production in glomeruli of diabetic rats.

Table 1 shows the expression of TGF-β1 mRNA and biglycan mRNA in glomeruli. Northern blotting analysis was performed with RNA prepared from glomeruli isolated from animals in each group as described in Figure 1. At three time points, each experimental group was divided into two equal groups that were used to conduct two independent experiments. Films were scanned using a laser densitometer. For quantitative comparison, the ratios of the density of the TGF-β1 and biglycan mRNA bands to that of the control glyceraldehyde phosphate dehydrogenase (GAPDH) mRNA band in the same lane were calculated. Results were then expressed as the relative changes of the ratios when the ratios of the normal control animals (C) were taken as 1.0. Values are means of two experiments.

These results further suggest a parallel between matrix expansion in the diabetic glomerulus and the repair process following tissue injury such as wounding. In both processes, there is an early expression of TGF-β mRNA and TGF-β protein associated with increased local production of extracellular matrix. Matrix components induced by TGF-β, fibronectin EDA+ and tenascin are preferentially expressed in healing wounds and in the diabetic glomerulus. Type III collagen is not found in normal glomeruli, but appears in diabetic glomeruli and in healing wounds. A key feature of wound repair is the necessity to terminate the process to avoid excessive scarring.

In patients with diabetes, kidney involvement occurs after 10 to 20 years of diabetes and progresses until the glomeruli are functionally obliterated by extracellular matrix as described in Knowles, Kidney Int. Suppl. 1:S2 (1974); Dorman et al., Diabetes 33:271 (1984); and Mauer et al., J. Clin. Invest. 74:1143 (1984). In the streptozotocin model of diabetes six or more months are required before there are glomerular abnormalities visible in the light microscope, a period comparable to 20 or more human years. Thus, the development of glomerulosclerosis in patients and experimental animals with diabetes suggests a chronic failure to regulate or terminate the process of tissue repair, possibly involving the continued activity of TGF-β. The stimulus to tissue repair in diabetes is likely to be chronic, episodic hyperglycemia which occurs despite insulin therapy. Hyperglycemia is known to cause numerous organ-specific and systemic alterations such as hemodynamic changes in the kidney and elevated blood levels of glycosylated proteins.

Composition useful in the methods of the present invention contain the agents that suppress the activity of TGF-β as previously identified together with insulin and a carrier. Pharmaceutically acceptable carriers include, for example, hyaluronic acid and aqueous solutions such as bicarbonate buffers, phosphate buffers, Ringer's solution and physiological saline supplemented with 5% dextrose or human serum albumin, if desired. Other pharmaceutical carriers known to those skilled in the art are also contemplated.

To show the relevance of the results obtained in the diabetic rat to human diabetic nephropathy, six cases of diabetic glomerulosclerosis (the end stage of diabetic nephropathy), three normal controls, and six disease controls consisting of three cases of minimal change disease and three cases of thin basement membrane disease were examined. The control diseases were chosen because they are glomerular disorders that produce proteinuria and/or hematuria but are known to rarely, if ever, to lead to glomerulosclerosis. All of the glomeruli in the six cases of diabetic glomerulosclerosis were strongly positive for TGF-β1 and fibronectin EDA+ as in the two cases shown in Figures 5 and 6. Due to limited quantity of tissue, the study was confined to these two markers. In contrast, each of the normal and disease controls were negative or showed only trace staining (Figures 5 and 6).

These findings establish a parallel between glomerular matrix expansion in diabetic nephropathy and experimental glomerulonephritis and a link between both diseases and the repair process following tissue injury such as wounding. In both there is early expression of TGF-β mRNA and TGF-β protein associated with increased local production of extracellular matrix. Matrix components induced by TGF-β, fibronectin EDA+ and tenascin, are preferentially expressed in healing wounds and, as shown in this study, in the diabetic rat glomerulus. A key feature of wound repair is the necessity to terminate the process to avoid excessive scarring. The development of glomerulosclerosis in patients with diabetes suggests a chronic failure to regulate or terminate the process of tissue repair resulting in continued activity of TGF-β and contrasts with our findings in the model of glomerulonephritis where TGF-β expression is transient and the disease is reversible. In patients with diabetes, kidney involvement occurs after ten or more years of diabetes and progresses until the glomeruli are functionally obliterated by extracellular matrix. In the streptozotocin model of diabetes several months are required before there are visible glomerular abnormalities, a period comparable to ten or more human years.

The following examples are intended to illustrate the invention.

### EXAMPLE I

### DIFFERENTIAL EFFECTS OF TRANSFORMING GROWTH FACTOR β ON EXTRACELLULAR MATRIX PRODUCTION BY MESANGIAL CELLS AND GLOMERULAR EPITHELIAL CELLS

### A. Growth Factors and Antibodies

Porcine TGF-β1 was obtained from R&D Systems, Inc. (Minneapolis, MN). Human platelet-derived growth factor (PDGF) and recombinant human interleukin-1 (IL-1), alpha and beta, were from Collaborative Research, Inc. (Bedford, MA). Recombinant human tumor necrosis factor (TNF) was obtained from Amgen (Thousand Oaks, CA). Goat anti-human type I and III collagen antibodies were purchased from Southern Biotechnology Associates, Inc. (Birmingham, AL). Rabbit anti-mouse fibronectin and types I, III, IV and VI collagen and production of anti-rat laminin antibodies have been described. Briefly, rabbit polyclonal antibodies were produced by injecting New Zealand rabbits with each purified protein emulsified in Complete Freund's Adjuvant subcutaneously. The initial immunization was followed three weeks later with sequential weekly injections of purified protein emulsified in Incomplete Freund's Adjuvant intramuscularly. The secondary injections were accompanied by a bleed of 50 ml from an ear artery. The blood was coagulated and the sera was collected by centrifugation. Monoclonal antibody to rat Thy-1 was obtained from Accurate Chemicals (Westbury, N.Y.) and goat anti-human factor VIII from Miles Laboratory Inc. (Kankokee, IL).

Rabbit anti-rat Fx1A antibody was produced by immunization with FX1A prepared from the kidney of Sprague-Dawley rats according to the method of Edgington. Briefly, rabbits were immunized as described above with a preparation enriched in brush border of the proximal renal tubules that was isolated by ultracentrifugation. FITC and alkaline phosphatase labeled or anti-rabbit IgG and anti-goat IgG were obtained from Cappel (Melvern, PA).

### B. Cell Culture

Glomerular epithelial cells and mesangial cells were obtained from outgrowth of intact glomeruli obtained from 6 to 8 week old Sprague-Dawley rats. Isolation and culture of mesangial cells was performed as follows.

Male Sprague/Dawley rats (100-140 grams, 2 rats per isolation) were anesthetized using ketamine (10 mg/100 g rat) and Rompum (0.5 mg/rat) and sterilized using betadine. The kidneys were exposed surgically and perfused using phosphate-buffered saline. The kidneys were then removed and placed in sterilized iced PBS.

The kidneys were bisected and the cortex was removed surgically. The remaining tissue was minced into 1 mm³ pieces using a scalpel. The minced kidney tissue was then passed through a series of sieves (149 mesh and 105 mesh) using continuous washing with PBS. The material passing through the 105 mesh sieve was then collected on a 74 mesh sieve. This represents the glomerular fraction.

This fraction was centrifuged for 10 minutes in a clinical centrifuge and the pellet was resuspended in RPMI 1640 media (Cell-Gro, Washington, D.C.) containing 20% fetal bovine serum, 0.66 units/ml insulin, and antibiotics. The glomeruli were plated at 2 kidney equivalents in 12 ml media in a T75 (75 cm²) flask. The mesangial cells grow out from the attached glomeruli after approximately 3 weeks of culture. Fresh media is added every 3-4 days during this culture period.

To isolate epithelial cells, intact glomeruli were placed in flasks coated with Vitrogen™ (Collagen Corporation, Palo Alto, CA). The growth medium was RPMI 1640 (Cell-Gro, Washington, D.C.) supplemented with 20% heat-inactivated fetal calf serum (FCS) (Hyclone, Logan, UT), 50 U/ml penicillin, 100 µg/ml streptomycin, 0.66 U/ml insulin and 300 mg/ml L-glutamine. After 7 days, outgrowing cells were detached with 0.025% trypsin-0.5M EDTA (Flow Labs, McLean, VA). Glomerular debris was removed by passing the material through a 30-mesh screen. A highly enriched epithelial cell population was produced by using an indirect panning technique. Specifically, glomeruli were isolated as described above and plated on tissue culture dishes pre-coated with rat tail collagen type I at 0.15 mg/ml. The dishes were coated for 1 hour at room temperature, then washed 2 times with PBS. After 1 week of culture, the outgrowth of cells (primarily epithelial cells) and glomeruli were trypsinized using 0.05% Trypsin-0.53 mM EDTA. The cell suspension was passed through a 30 mesh to remove glomerular pieces. The remaining cell suspension was plated on a dish previously coated with a monoclonal antibody against the Thy 1 antigen. Only mesangial cells will bind to the Thy 1 antibody because they express the antigen, whereas the epithelial cells do not and thus remain in suspension. This procedure preferentially eliminated mesangial cells. Plates were coated with antibody (approximately 100 µg/ml) at 4°C for 24 hours. The incubation of cells on the anti-Thy 1-coated dish was for 1 hour at 37°C. The unbound cells were collected and replated. Glomerular cells were added to 60 X 15 mm polystyrene plates, which were precoated with mouse anti-Thy-1 antibody by 12 hour incubation at 4°C. Nonadherent cells were removed and placed in 6-well plates precoated with laminin (Collaborative Research, Bedford, MA).

Glomerular epithelial cells were identified by: 1) characteristic polygonal morphology; 2) uniform staining with antibody to FX1A (Heymann antigen); 3) sensitivity to aminonucleoside of puromycin; and 4) no staining with anti-Thy-1 or anti-Factor VIII antibody. Contamination of glomerular epithelial cells by proximal tubular cells was examined by alkaline phosphatase staining, and less than 2% of the cells were alkaline phosphatase positive. In contrast, mesangial cells were identified by 1) typical stellate appearance; 2) uniform staining with anti-Thy-1 antibody; 3) no staining with anti-FX1A or anti-Factor VIII antibody; and 4) no sensitivity to aminonucleoside of puromycin.

### C. Biosynthetic Radiolabeling

Glomerular epithelial and mesangial cells were added to regular or laminin-coated 6-well multiwell plates at a concentration of 5 X 10⁵ cells per well and cultured in serum-free RPMI 1640 medium for 24 hours to arrest cell proliferation. Non-adherent cells were removed by washing with phosphate buffered saline (PBS). Serum and antibiotic-free RPMI 1640 was added as a low sulfate medium for ³⁵S sulfate labeling and RPMI 1640 without methionine (Flow Labs, McLean, VA) for ³⁵S methionine labeling. Growth factors were added to the media for 48 hours at the following concentrations: TGF-β (25 ng/ml), PDGF (2 U/ml), IL-1α (5 U/ml), IL-1β (5 U/ml) and TNF (500 U/ml). Eighteen hours prior to termination of the experiment, ³⁵S methionine (100 µCi/ml), to label proteins or ³⁵S sulfate (200 µCi/ml) to label proteoglycans were added to the cultures.

Isotopes were purchased from New England Nuclear (Boston, MA). Conditioned media and cell layers were harvested and processed as described above. Proliferation of cells was examined by the uptake of ³H-thymidine. Cells were added to laminin-coated 12 well plates at a concentration of 2 X 10⁵/well. After 24 hours in serum free medium, 25 µl of a 10 µCi/ml solution of ³H-thymidine was added to each well. Incubation was carried out for 24 hours. The culture medium was discarded, cell layers were washed 2 times with 5% TCA solution, and incubated for 15 minutes with 700 µl of 6 N HCl solution. Incorporation of ³H-thymidione was measured by counting each cell layer in a liquid scintillation counter (Beckman, Irvine, CA) and corrected with protein contents of each cell layer which were measured by a BCA protein assay kit (Pierce, Rockford, IL).

### D. Identification of Matrix Molecules

Matrix glycoproteins were identified by immunoprecipitation and proteoglycan by enzyme digestion. Immunoprecipitations were performed by adding 100 µl of antiserum to 500 µl of conditioned medium or 300 µl of cell extract collected from duplicate wells in the presence or absence of added growth factors. In duplicate wells, cells were detached and counted to ensure uniformity of cell number. Preimmune serum was used in parallel control experiments. The samples were incubated overnight at 4°C with mixing in 4 ml conical tubes precoated with bovine serum albumin (BSA). Protein-A-Sepharose beads (Sigma, St. Louis, MO) were preincubated with fresh RPMI for 60 minutes at 22°C. To precipitate the antigen-antibody complexes, 50 µl of suspended protein-A-Sepharose was added to the samples, and mixed at 4°C for 120 minutes. The samples were centrifuged for 10 minutes at 2000 x G and the supernatant removed. The pellets were washed 10 times with 1 ml of ice cold PBS containing 0.5 M NaCl, 0.1% Triton X-100 pH 7.4. Finally, the pellets were washed with ice cold PBS, transferred to new tubes, recentrifuged, and washed 3 times with PBS. The pellets were dissolved in 40 µl of SDS-PAGE sample buffer containing 3% SDS and 10% β mercaptoethanol (Sigma, St. Louis, MO) and boiled for 5 minutes.

Digestion with glycosaminoglycan-degrading enzymes was used to determine the type of proteoglycans that were regulated by TGF-β. The digestion was performed on conditioned media after biosynthetic labeling. Aliquots of medium (25 µl) were mixed with 100 milliunits of chondroitinase ABC or chondroitinase AC both in 100 mM Tris-HCl, pH 7.5, 10 mM calcium acetate, 2 mg/ml BSA or 100 milliunits of heparinase II in 50 mM Tris-HCl, pH 7.4, 1 mM calcium chloride, 5 mM calcium acetate. All samples also received 1 mM PMSF, 5 mM benzamidine, 100 µg/ml soy bean trypsin inhibitor, 10 µg/ml leupeptin and 10 µg/ml antipain. All materials were obtained from Sigma Chemical Co. ( St. Louis, MO). Chondroitinase-containing mixtures were incubated at 37°C for 1.5 hours. At termination samples were prepared for SDS-PAGE.

Large proteoglycans were characterized by gel filtration. ³⁵S sulfate labeled fractions from a sepharose CL-6B column were dialyzed against distilled water and lyophilized. Samples were dissolved in 2 ml of 0.1M sodium acetate 0.1M tris, pH 7.3, containing protease inhibitors described above. Then 0.2 ml of chondroitinase ABC solution (1.25 units/ml) was added to 1.8 ml of sample digestion carried out for 24 hours at 37°C. Samples were then fractionated on a CL-6B column, dialyzed against distilled water, lyophilized and treated with nitrous acid under low pH (1.0) conditions. After treatment with nitrous acid, samples were rechromatographed on a CL-6B column.

To quantitate the increased production of proteoglycans induced by TGF-β, three ml of conditioned medium were placed on a sepharose CL-6B column (1.3 X 100 cm). The column was eluted with 4M urea, 0.15M NaCl, 50 mM Tris HCl, pH 7.2, 0.1% (v/V) Triton X-100 at a flow rate of 20 ml/hour, and fractions of 3 ml/tube were collected. The radioactivity in each effluent fraction was determined by a Beckman LS 2800 liquid scintillation counter.

### E. Electronhoretic Technique

Samples were analyzed by SDS-PAGE with fluorography and densitometry as described in Example II below.

### F. Results

The results of the foregoing studies demonstrate that TGF-β regulates the production of biglycan, fibronectin and type IV collagen by the epithelial cells, while TGF-β only affects proteoglycan production by cultured mesangial cells. The biosynthetic profile of matrix production by epithelial cells, regulated by TGF-β, is thus different from that of mesangial cells. More specifically, these results demonstrate that release of TGF-β in the glomerulus leads to mesangial matrix accumulation and an increase in production of matrix molecules that form the glomerular basement membrane. The results are summarized in Table 2.

**TABLE 2**

| TGF-β Induction of Extracellular Matrix by Glomerular Cells in Culture | | | | |
|---|---|---|---|---|
| Matrix Components | Mesangial Cell | | Epithelial Cell | |
| | Medium | Monolayer | Medium | Monolayer |
| | | | | |

| Proteoglycans | | | | |
|---|---|---|---|---|
| Biglycan | ↑↑↑↑ | ↑ | ↑↑↑↑ | 0 |
| Decorin | ↑↑↑↑ | ↑ | ↑ | 0 |
| Heparan Sulfate | 0 | 0 | 0 | 0 |
| Fibronectin | 0 | ↑ | ↑↑↑↑ | ↑↑↑↑ |
| Laminin | 0 | 0 | ↑ | ↑↑ |

| Collagen | | | | |
|---|---|---|---|---|
| Type I | 0 | 0 | NP | NP |
| Type III | 0 | 0 | NP | NP |
| Type IV | 0 | 0 | ↑↑↑ | ↑↑↑ |
| Type VI | 0 | 0 | NP | NP |
| ↑ = induction, 0 = no effect, NP = component not produced | | | | |

### EXAMPLE II

### A. INDUCTION OF DIABETES IN RATS

Diabetes was induced in male Sprague-Dawley rats, weighing 150 grams by a single IV injection of streptozotocin (Sigma Chemical Co., St. Louis, MO), at 6.5 mg/100 g body weight in citrate buffer as described in Velasquez et al., FASEB 4:2850 (1990) and Mauer et al., Diabetes 25:850 (1976), both incorporated herein by reference. Forty eight diabetic rats with blood glucose levels >300 mg/dL were separated into two groups. The first diabetic group (D) received no treatment, the second group (DI) was treated with a daily subcutaneous injection of 3.5 U of NPH insulin (Eli Lilly, Indianapolis, IN). Forty eight age-matched male rats served as controls and were divided into groups that received no insulin (C) or were treated with the same insulin regimen (CI) as the diabetic rats.

Blood glucose levels were measured in each group after 10 weeks at 8:00 a.m. following an overnight fast and the values in mg/dL are reported in Table 3. The values are mean ± standard deviation.

**TABLE 3**

| Blood Glucose Levels | |
|---|---|
| D | 456 ± 38 |
| DI | 329 ± 31 |
| C | 122 ± 10 |
| CI | 137 ± 15 |

After 1, 6, 15 and 40 weeks, 6 rats in each group were anesthetized with 1 M ketamine HCL, 10 mg/100 g body weight and xylazine 0.5 mg/100 g body weight for removal of kidneys. Diabetic rats without insulin (D) did not survive beyond 37 weeks and are thus absent from the 40 week analysis. For all techniques, kidneys were perfused in situ with cold phosphate buffered saline (PBS), pH 7.4, and then excised. Pieces of cortex were removed for immunohistologic examination and glomeruli were isolated from the remaining tissue for mRNA detection as described in Okuda et al., J. Clin. Invest. 86:453 (1990), which is incorporated herein by reference. Differences between groups in immunofluorescence scoring of matrix components and TGF-β1 positive cells were analyzed by t test.

### B. PREPARATION AND DETECTION OF TOTAL RNA

Total RNA was prepared by lysis of isolated glomeruli (8-9 x 10⁴ cells) in 3-4 mls of guanidine isothiocyanate (GITC) solution (4 M GITC: 0.5% w/v N-isoryl-sarcosine, 0.1% anti-foam A, 0.1 M β-mercaptoethanol, 25 mM Tris-HCl, pH 7.0) containing 1% 2-mercaptoethanol and 0.5% lauryl sarcosyl. The lysate was subjected to ultracentrifugation 150,000 x g on a cesium chloride density gradient cushion. The RNA was recovered from the bottom layer of the tube after centrifugation. Forty micrograms of RNA were pooled in each group at each time point and electrophoresed in 1% agarose gels containing 2.2 M formaldehyde and 0.2 M MOPS (Sigma Chemical Co., St. Louis, MO), pH 7.0 and transferred to a 0.2 micron nylon membrane (ICN Radiochemicals, Inc., Irvine, CA) overnight by capillary blotting.

Equal amounts of RNA (40 µg) were loaded on the basis of absorbance at 260 nm. The membranes were prehybridized for 5 hours at 37°C in 5x SSC (20x SSC: 175.3 g NaCl, 88.2 Na citrate, pH 7.0 with 10 N NaOH to a final volume of 1 liter), 5x Denhardt's solution (50x solution: 5 g ficoll, 5 g polyvinylpyrrolidone, 5g BSA, H₂ (0 to 500 ml), 50 mM sodium phosphate, pH 6.5, 0.1% SDS, 250 µg/ml of sonicated denatured salmon sperm DNA (Sigma Chemical Co., St. Louis, MO) and 50% formamide.

To obtain a mouse cDNA probe, a 280 bp PvuII fragment was subcloned into HincII digested pBluescript SKII+ (Stratagene, La Jolla, CA) from a mouse TGF-β1 cDNA obtained from Dr. R. Derynck and described in Derynck et al., J. Biol. Chem. 261:4377 (1986), which is incorporated herein by reference. The orientation of the insert, which encoded amino acids 298-390 of mature TGF-β1, was determined and the plasmid was purified by cesium chloride density centrifugation as described in Kondaiah et al., J. Biol. Chem. 263:18313 (1988), which is incorporated herein by reference.

The mouse cDNA probe, a rat GAPDH cDNA probe, provided by Dr. M. B. Sporn, National Institute of Health (NIH), and a human biglycan cDNA probe (plasmid p16) provided by Dr. L. W. Fisher, NIH, were labeled with ³²P-CTP (cytosine triphosphate) by the random primer method (Boehringer Mannheim, Indianapolis, IN) and hybridized at 42°C overnight. The membranes were washed three times in 2X SSC, 0.1% SDS at room temperature for 5 minutes and three times in 0.1X SSC, 0.1% SDS at 50°C for 15 minutes. Autoradiography was performed by standard methods. Films were scanned using a laser densitometer (LKB, Bromma, Sweden). For quantitative comparison, the ratios of the density of the TGF-β1 and biglycan mRNA bands to that of the GAPDH mRNA band in the same gel lane were calculated. Results were then expressed as the relative changes of the ratios when the ratios of the normal control animals (C) were taken as 1.0.

The results of this analysis are shown in Figure 1 and summarized in Table 1. When standardized by comparison to the level of GAPDH mRNA, there is a 3.4 fold increase in TGF-β1 mRNA and a 2.3 fold increase in biglycan mRNA in the diabetic rats not treated with insulin. Diabetic rats treated with insulin showed less of an elevation of TGF-β1 and biglycan mRNA.

### C. DETECTION OF TGF-β IN DIABETIC GLOMERULI

Tissues of glomeruli were snap-frozen in liquid nitrogen, fixed in acetone, and 4 µm sections were stained with an antibody (50 µg/ml) made against a synthetic peptide corresponding to the first 30 amino acids of mature TGF-β1. This antibody (anti-LC) (gift from Drs. K.C. Flanders and M.B. Sporn, National Institutes of Health) stains intracellular TGF-β1. The anti-LC antibody is published in Flanders et al., Biochem. 27:739 (1988), the teachings of which are incorporated herein by reference. Fluorescein isothiocyanate-conjugated antiserum (x500) to rabbit IgG or lissamine rhodamine donkey F(ab')2 anti-rabbit IgG (Jackson Immunology Lab, West Grove, PA) was used as the second antibody at a dilution of 1:300.

The TGF-β1 positive cells were identified by a double-staining immunofluorescence technique employing fluorescein and rhodamine conjugated secondary antibodies with primary staining using monoclonal antibodies to ED1 (monocyte/macrophages), OX19 (lymphocytes), and vimentin, desmin, and α-smooth muscle actin (Jackson Immunoresearch Lab). The number of TGF-β1 positive cells were counted in 20 glomeruli selected at random in coded sections prepared from each of six animals in the control and diabetic groups.

Staining of a glomerulus from a diabetic rat, not treated with insulin, 15 weeks after induction of diabetes shows a striking increase in the number of cells positive for TGF-β1. In the diabetic glomeruli, there were significantly more positive cells per glomerulus, *32±2 versus 25±1, (values are mean ± s.d., *p < 0.001) compared to glomeruli from the control rats.

### D. QUANTITATION OF FIBRONECTIN EDA+ AND TENASCIN DEPOSITION IN GLOMERULI OF DIABETIC RATS

Tissues for immunofluorescence were prepared as described. Mouse monoclonal antibody prepared against human fibronectin EDA+ was kindly provided by Dr. L. Zardi, Istituto Nazionale per la Ricerca sul Cancro, Italy and is described in Balza et al., FEBS Letters 228:42 (1988) and Sekiguichi et al., J. Biol. Chem. 260:5104-5114 1985), both of which are incorporated herein by reference. Rabbit anti-human tenascin was obtained from Telios Pharmaceuticals Inc. (La Jolla, CA). Tenascin was isolated from human glioblastoma U251 cells as described in Bourdon et al., Cancer Res. 43:2796-2805 (1983), which is incorporated herein by reference. Fluorescein isothiocyanate anti-rabbit IgG and rat F(ab')2 anti-mouse IgG (both obtained from Jackson Immunology Lab, West Grove, PA) were used as second antibodies.

In immunofluorescence (IF) staining for tenascin, 4 µm tissue sections were immersed in acetone for 4-5 minutes. Without drying, the fixed sections were immersed in PBS, pH 7.4 for about 10 minutes at room temperature. The sections were then mildly dried using a fan for about 5 minutes until only a few droplets of water remained on the slide. Rabbit polyclonal anti-human tenascin antibody (Telios Pharmaceuticals, La Jolla, CA) was diluted 1:15 to 1:20 with PBS containing 0.01% sodium aside. About 10 to 15 µl of the PBS solution was applied to the tissue and thereafter incubated for about 45 minutes at 37°C in a moist chamber. The slide was then tilted to allow a PBS wash to gently run over it without applying the wash directly onto the area. The sections were then immersed in PBS three times for 5 minutes each at room temperature with shaking. The sections were then dried with a fan. The FITC-conjugated affinity purified donkey f(ab')₂ anti-rabbit IgG (Jackson Immuno. Lab, 711-096-132, #14620) diluted 1:400 with PBS containing 0.01% sodium aside was added to the slides. The slides were washed with PBS three times as above. The cover glass was mounted over the tissue using Bartels buffered glycerol mounting medium FA (Baxter Laboratories).

The IF staining for fibronection EDA+ followed the above procedure. Neat mouse monoclonal anti-human fibronection EDA was used as a primary antibody and FITC-conjugated affinity purified F(ab')₂ rat anti-mouse IgG (Jackson Immunoresearch Lab. #16381) diluted 1:30 with PBS containing 0.01% sodium azide was used as the secondary antibody.

The results of the quantitation assays are shown in Figure 2. Immunofluorescence staining was scored using a 0 to 4 scale (0 = no staining, 4 = maximal strong staining) in 20 glomeruli selected at random in coded sections prepared from six animals in each experimental group. At each time point there is a striking increase in deposition of the two matrix components in the diabetic animals.

In the detection of fibronectin EDA+ deposition in control and diabetic glomeruli with immunofluorescence micrographs of glomeruli stained with anti-fibronectin EDA+ antibody, the glomerulus of a normal control rat, treated with insulin for 40 weeks shows faint staining, while the glomerulus from a rat with diabetes, treated with insulin for 40 weeks shows a marked increase in staining.

### EXAMPLE III

### INHIBITION OF PROTEOGLYCAN SYNTHESIS WITH AN ARG-GLY-ASP CONTAINING PEPTIDE

Mesangial cells were obtained from intact glomeruli of 4 to 6 week old Sprague-Dawley rats according to the method of Harper, et al., Kidney International 26:875 (1984), which is incorporated herein by reference. The growth medium used was RPMI 1640 (Cell-Gro, Washington, D.C.) supplemented with 20% heat-inactivated fetal calf serum (FCS) (Hyclone, Logan, UT), 50 U/ml penicillin, 100 µg/ml streptomycin, 0.66 U/ml insulin, and 300 mg/ml L-glutamine. Between day 15 to 20, primary cultures were detached with a solution of 0.025% trypsin - 0.5 mM EDTA (Flow Labs, McLean, VA) and 2 x 10⁶ cells were added to flasks. The cells were passed every 7 days and all experiments were performed on cells between passages 3 and 7.

Rat mesangial cells were grown to subconfluency in 6-well multiplates. The cultures were made serum free for 24 hours and TGF-β₁ was added at 25 ng/ml along with Gly-Arg-Gly-Asp-Ser-Pro (GRGDSP) at 0.3, 0.1, 0.03, 0.01 or 0.003 mg/ml, or Gly-Arg-Gly-Glu-Ser-Pro (GRGESP) at 0.3 mg/ml. The peptides were synthesized as described in Pierschbacher and Ruoslahti, J. Biol. Chem., 292:1794-1798 (1987) which is incorporated by reference herein, on an Applied Biosystems Peptide Synthesizer according to the manufacturer's protocol. Thirty hours later the cultures were metabolically labeled with ³⁵S-sulfate and 18 hours afterward the conditioned media were analyzed by SDS-PAGE with fluorography. The fluorograms were scanned with a laser densitometer and the following represent relative densitometric units for the proteoglycan bands. Control 1.9; TGF-β₁ 4.5; TGF-β₁+GRGDS 0.3 mg/ml, 1.3; 0.1 mg/ml, 2.4; 0.03 mg/ml, 2.6; 0.01 mg/ml, 3.9; 0.003 mg/ml, 4.0; and TGF-β₁+GRGESP 0.3 mg/ml, 4.3.

These data show a dose response effect of higher doses of GRGDSP causing inhibition of the TGF-β₁-induced proteoglycan production with no effect of the control peptide GRGESP.

### EXAMPLE IV

Kidney tissue from kidney biopsy or nephrectomy samples obtained from patients undergoing diagnostic or surgical evaluation at the University of Utah Medical Center was studied. The analysis included six cases of diabetic nephropathy, three cases of minimal change disease, three cases of thin basement membrane disease, and three normal kidneys. Four or more glomeruli were present in each sample, and the diagnosis was established in each case using conventional clinical and pathological criteria by physicians not involved in the current study. Frozen tissue was studied for the presence of TGF-β1 protein and fibronectin EDA+ by immunofluorescence microscopy as described in Example II for the experimental model. The human and animal studies were approved by the Institutional Review Board of the University of Utah School of Medicine.

All of the glomeruli in the six cases of diabetic glomerulosclerosis were strongly positive for TGF-β1 and fibronectin EDA+ as in the two cases shown in Figures 5 and 6. Due to limited quantity of tissue, the study was confined to these two markers. In contrast, each of the normal and disease controls were negative or showed only trace staining (Figures 5 and 6).

### EXAMPLE V

### NEUTRALIZATION OF THE ACTIVITY OF TGF-B1, 2 AND 3 BY RECOMBINANT HUMAN DECORIN

### A. Reagents

Purified human TGF-β1 and 2 were obtained from R & D Systems (Minneapolis, MN), and TGF-β3 was a gift from Drs. A. Roberts and M. Sporn, National Institutes of Health.

Recombinant human decorin was prepared from the culture media of Chinese hamster ovary cell line clone 62 as described by Yamaguchi et al., supra, hereby incorporated by reference. Decorin was purified by a modified procedure combining ion exchange on Q-Sepharose and hydrophobic chromatography on octyl-Sepharose as described by Choi et al., J. Biol. Chem. 264: 2876(1989), incorporated herein by reference. After elution of decorin from the octyl-Sepharose column by 4 M guanidine HCl, the preparation was dialyzed against PBS, pH 7.4, and the decorin concentrations were adjusted to 0.9 mg per ml.

Ovalbumin and bovine serum albumin were obtained from Sigma (St. Louis, MO) and were prepared under the same conditions that were used in the purification of decorin.

### B. Cell Culture

A mink lung cell assay that measures growth inhibition was used to assay the ability of recombinant decorin to neutralize the activity of the TGF-β isoforms. [³H]Thymidine incorporation was determined in the presence of TGF-β and increasing concentrations of decorin (o) or bovine serum albumin (BSA) (●) as described by Yamaguchi et al., supra. TGF-βs were added at the concentrations that inhibited the incorporation of [³H]thymidine by 50%; these concentrations were 0.2 ng/ml, 0.1 ng/ml and 0.05 ng/ml for TGF-β1, TGF-β2, and TGF-β3, respectively. The data shown in Figure 7 represent percent neutralization of TGF-β1-induced growth inhibition. [³H]Thymidine incorporation in the presence of neither TGF-β nor decorin is defined as 100% (125,000 cpm for TGF-β1 and TGF-β3; 107,000 cpm for TGF-β2). Incorporation in the presence of TGF-β but not decorin is defined as 0% (57,000 cpm for TGF-β1; 78,000 cpm for TGF-β2; 59,000 cpm for TGF-β3). Each point represents the mean of results from duplicate samples.

As shown in Figure 7, decorin neutralized the growth inhibitory activity of each of the three TGF-βs.

The anti-TGF-β1 serum used previously to ameliorate glomerulonephritis, as described by Border et al., Nature 346:371 (1990), was also examined. This antiserum neutralizes TGF-β1 but not TGF-β2 nor 3 (data not shown). Thus, decorin has a broader binding reactivity against the TGF-βs than does the anti-TGF-β1.

### EXAMPLE VI

### INTRAVENOUS ADMINISTRATION OF RECOMBINANT HUMAN DECORIN

Intravenous administration of recombinant human decorin and decorin purified from bovine tissues was used to test for the ability of the proteogylcan to suppress matrix accumulation in glomerulonephritic rat kidneys. The amount of fibronectin present in glomeruli was used quantitatively to assess the ability of decorin to block matrix deposition in glomerulonephritis.

### A. Protocol

Glomerulonephritis was induced in Sprague-Dawley rats (4-6 wk old) by intravenous injection of antithymocyte serum as described by Okuda, et al., supra. Age matched rats served as normal controls. Recombinant human decorin, prepared as described in Example V.A., and bovine decorin were tested in separate but identical experiments according to the following protocol.

Eight rats were divided into four groups of two each and injected with antithymocyte serum. One hour later treatment was begun with a single intravenous injection of 0.45 mg of recombinant human decorin or bovine decorin in 0.5 ml of PBS or, as control, 0.5 ml PBS alone (rats were restrained but not anesthetized for the injections). Bovine decorin was isolated from articular cartilage by extraction for 24 hours with 4M guanidine HCl and protease inhibitors and then purified as described in Example V.A. Treatment was continued with a single daily injection of decorin or PBS as follows: Decorin was administered to Group 1 for two days, Group 2 for four days and Group 3 for six days. Group 4 received PBS alone for six days. When decorin was discontinued in Groups 1 and 2, PBS was substituted so that all animals received six daily injections. All treatment was stopped on day 7, a 24 h urine was collected, after which the animals were sacrificed and the kidneys were removed.

An experiment was conducted, according to the protocol described above, to test the control preparations as follows: Eight rats were injected with antithymocyte serum, divided into groups of two rats each, and injected daily for six days with 0.5 ml of PBS alone or PBS containing aggrecan, ovalbumin, bovine serum albumin. The carbohydrate content of aggrecan and decorin were determined and aggrecan in PBS was injected at the same carbohydrate concentration as decorin. An additional four rats received a single injection of PBS instead of antithymocyte serum and served as normal controls. All animals were sacrificed following a 24 h urine collection as described above and urinary protein and creatinine were measured as described by Okuda et al., supra.

For all techniques kidneys were perfused in situ with cold PBS and then excised. Kidney tissue was prepared for light and immunofluorescence microscopy as described by Okuda et al., supra, incorporated by reference herein.

### B. Fibronectin Staining

Fluorescein isothiocyanate-conjugated sheep anti-human fibronectin was from the Binding Site, Inc. (San Diego, CA). Immunofluorescence staining for fibronectin was scored without knowledge of the source of the tissue using a 0 to 4 scale (0 = no staining, 4 = maximal strong staining) in 20 glomeruli selected at random in coded sections prepared from each animal. Differences between groups were analyzed by t test as described by Okuda et al., supra.

### C. EDA+ and Tenascin Staining

Glomeruli were stained with antibodies that detect EDA+ fibronectin and tenascin; these matrix components are relatively specifically induced by TGF-β. Mouse monoclonal antibody prepared against human fibronectin containing extra domain A (EDA+), was kindly provided by Dr. L. Zardi, Instituto Nazionale per la Ricerca sul Cancro, Italy. Rabbit anti-human tenascin was obtained from Telios Pharmaceuticals, Inc. (La Jolla, CA). Fluorescein isothiocyanate-conjugated rat F(ab')2 anti-mouse IgG and donkey F(ab')2 anti-rabbit IgG were used as second antibodies (Jackson Immunoresearch Lab, West Grove, PA). The technique of matrix scoring and the method of statistical analysis for EDA+ fibronectin and tenascin staining are the same as for fibronectin, which is described above.

### D. Results

Figure 8 shows that four or six daily injections of recombinant decorin or bovine decorin suppressed fibronectin deposition to a level not significantly different from that found in the normal rats. Control injections consisted of buffer alone, a different proteoglycan and two proteins. A cartilage proteoglycan, aggregan, was used as a negatively charged molecule to control for the glycosaminoglycan chain of decorin; aggregan does not inhibit TGF-β, as described by Yamaguchi et al., supra. Ovalbumin was used as a control for the core protein of decorin because both proteins are approximately the same molecular size. Serum albumin was used as an unrelated protein control. Figure 8 indicates that the proteoglycan and protein controls were indistinguishable from glomerulonephritic rats injected with buffer alone. Two injections of decorin given early in the disease process also had no effect on the fibronectin accumulation.

As shown quantitatively in Figure 9, EDA+ fibronectin is only present in trace amounts in glomeruli of normal rats, and a similar finding was obtained for tenascin. The results show that both EDA+ fibronectin and tenascin were greatly increased in nephritic glomeruli in rats treated with no or two injections of decorin. However, as was the case with whole fibronectin, four or six injections of decorin greatly reduced the deposition of these two markers of TGF-B activity (Figure 9). Histological analysis of periodic acid-Schiff stained sections of the same kidneys showed a comparable effect of decorin in preventing the increase in glomerular extracellular matrix and associated glomerular enlargement that occurs in the disease.

Decorin given for four or six days also suppressed the development of proteinuria. The ratio or urinary protein concentration to the urinary concentration of creatinine was used to assess proteinuria because this method minimizes the influence of differences in urinary volume on the protein excretion values, as described by Ginsberg et al., supra. Mean values for normals, disease controls, low dose decorin (two injections) and high dose decorin-treated rats (four and six injections) were respective 0.7, 15.2, 14.8 and 2.4 (p <0.01 for high dose decorin rats compared to disease controls).

These results show a dramatic effect of decorin in preventing the deposition of extracellular matrix in injured glomeruli from nephritic rats. Previously it was shown that the accumulation of extracellular matrix in glomerulonephritis is caused by overproduction of TGF-β (Okuda et al., supra, Border et al., (1990) supra). That matrix accumulation in this model was prevented by injection decorin demonstrates an in vivo activity of decorin in suppressing TGF-β.

## Claims

1. Use of decorin or biglycan for the preparation of a medicament for preventing or treating a pathology characterized by an accumulation of extracellular matrix in a tissue, wherein said pathology is diabetes-associated pathology.

2. The use of claim 1, wherein said pathology is diabetic nephropathy.

3. Use of insulin and decorin or biglycan for the preparation of a medicament for treating diabetic nephropathy.

4. A composition comprising (a) insulin and (b) decorin or biglycan

## Patentansprüche

1. Verwendung von Decorin oder Biglycan zur Herstellung eines Medikaments zur Verhinderung oder Behandlung einer Erkrankung, die durch eine Akkumulation extrazellulärer Matrix in einem Gewebe gekennzeichnet ist, wobei die Erkrankung eine diabetes-assoziierte Erkrankung ist.

2. Verwendung nach Anspruch 1, wobei die Erkrankung Diabetische Nephropathie ist.

3. Verwendung von Insulin und Decorin oder Biglycan zur Herstellung eines Medikaments zur Behandlung Diabetischer Nephropathie.

4. Zusammensetzung, die (a) Insulin und (b) Decorin oder Biglycan umfaßt.

## Revendications

1. Utilisation de décorine ou de biglycanne pour la préparation d'un médicament pour la prévention ou le traitement d'une pathologie caractérisée par une accumulation de matrice extracellulaire dans un tissu, dans laquelle ladite pathologie est une pathologie associée au diabète.

2. Utilisation selon la revendication 1, dans laquelle ladite pathologie est la néphropathie diabétique.

3. Utilisation d'insuline et de décorine au de biglycanne pour la préparation d'un médicament pour le traitement de la néphropathie diabétique.

4. Composition comprenant (a) de l'insuline et (b) de la décorine ou du biglycanne.
